# EUROPEAN PATENT APPLICATION

(11) **EP 2 893 880 A1**
(43) Date of publication of application: **15.07.2015**
(21) Application number: 14180322.1
(22) Date of filing: 08.08.2014
(51) Int. Cl.: A61B 8/00, A61B 8/08

(54) **Ultrasound diagnostic apparatus and method of operating the same**

(30) Priority: 08.01.2014 KR 20140002496
(71) Applicant: Samsung Medison Co., Ltd., Gangwon-do 250-870 (KR)
(72) Inventor: Park, Sung-wook, Gangwon-do (KR); Chang, Hyuk-jae, Seoul (KR); Chung, Nam-sik, Seoul (KR); Hong, Geu-ru, Seoul (KR); Song, Joo-hyun, Gangwon-do (KR); Shin, Sang-hoon, Seoul (KR); Lee, Bong-heon, Gangwon-do (KR); Lee, Jin-yong, Gangwon-do (KR); Lee, Hyun-jin, Seoul (KR)
(74) Representative: Schmid, Wolfgang

(57) **Abstract**

Disclosed are an ultrasound diagnostic apparatus and a method of operating the same. The method includes transmitting an ultrasound signal to an object to receive an echo signal corresponding to the ultrasound signal from the object, generating an ultrasound image, based on the received echo signal, detecting cross-sectional information indicating which cross-sectional surface of the object the generated ultrasound image shows, and displaying the ultrasound image and a cross-sectional information image corresponding to the detected cross-sectional information.

## Description

### RELATED APPLICATIONS

This application claims the benefit of Korean Patent Application No. 10-2014-0002496, filed on January 8, 2014, in the Korean Intellectual Property Office, the disclosure of which is incorporated herein in its entirety by reference.

### BACKGROUND

### 1. Field

One or more embodiments of the present invention relate to an ultrasound diagnostic apparatus and method of operating the same, and more particularly, to an ultrasound diagnostic apparatus and method of operating the same, which display a cross-sectional information image corresponding to an ultrasound image.

### 2. Description of the Related Art

Ultrasound diagnostic apparatuses irradiate an ultrasound signal, generated from a transducer of a probe, onto an object and receive information of an echo signal reflected from the object, thereby obtaining an image of an internal part of the object. In particular, ultrasound diagnostic apparatuses are used for the medical purpose of observing the inside of an object, detecting a foreign material, and assessing an injury. Ultrasound diagnostic apparatuses have stabilities higher than those of diagnostic apparatuses using X-rays, display an image in real time, and are safe because there is no exposure to radioactivity, and thus may be widely used along with other image diagnostic apparatuses.

Ultrasound diagnostic apparatuses may provide a brightness (B) mode in which a reflection coefficient of an ultrasound signal reflected from an object is shown as a two-dimensional (2D) image, a Doppler mode image in which an image of a moving object (particularly, blood flow) is shown by using the Doppler effect, and an elastic mode image in which a reaction difference between when compression is applied to an object and when compression is not applied to the object is expressed as an image.

### SUMMARY

One or more embodiments of the present invention include an ultrasound diagnostic apparatus and method of operating the same, which display a cross-sectional information image corresponding to an ultrasound image, thereby enabling a user to easily determine which cross-sectional surface of an object the ultrasound image shows.

Additional aspects will be set forth in part in the description which follows and, in part, will be apparent from the description, or may be learned by practice of the presented embodiments.

According to one or more embodiments of the present invention, a method of operating an ultrasound diagnostic apparatus includes: transmitting an ultrasound signal to an object to receive an echo signal corresponding to the ultrasound signal from the object; generating an ultrasound image, based on the received echo signal; detecting cross-sectional information indicating which cross-sectional surface of the object the generated ultrasound image shows; and displaying the ultrasound image and a cross-sectional information image corresponding to the detected cross-sectional information.

The method may further include mapping and storing the cross-sectional information image corresponding to the cross-sectional information.

The stored cross-sectional information image may be a cross-sectional image corresponding to the ultrasound image.

The cross-sectional information image may be an image in which a cross-sectional surface corresponding to the ultrasound image is displayed in a three-dimensional (3D) image of the object.

The detecting of cross-sectional information may include extracting at least one of a shape, length, width, and brightness value of a sub-object included in the ultrasound image and a position relationship with a peripheral sub-object to detect the cross-sectional information of the ultrasound image.

The method may further include: displaying a first pointer at a first position of the ultrasound image, based on a user input; and displaying a second pointer at a second position of the cross-sectional information image corresponding to a position of the first pointer.

The method may further include displaying names of a plurality of sub-objects included in the ultrasound image, based on the cross-sectional information image.

The ultrasound image may include first and second ultrasound images, the displaying of the ultrasound image may include displaying the first and second ultrasound images, and the displaying of a cross-sectional information image may include displaying a first cross-sectional surface corresponding to the first ultrasound image and a second cross-sectional surface corresponding to the second ultrasound image, in a three-dimensional (3D) image of the object.

The displaying of a cross-sectional information image may include displaying the first and second cross-sectional surfaces in different colors.

The method may further include selecting one of the first and second ultrasound images, based on a user input, wherein the displaying of a cross-sectional information image may include displaying a cross-sectional image which corresponds to the selected ultrasound image.

According to one or more embodiments of the present invention, an ultrasound diagnostic apparatus includes: an ultrasound transceiver that transmits an ultrasound signal to an object, and receives an echo signal corresponding to the ultrasound signal from the object; an image generating unit that generates an ultrasound image, based on the received echo signal; a cross-sectional information detecting unit that detects cross-sectional information indicating which cross-sectional surface of the object the generated ultrasound image shows; and a display unit that displays the ultrasound image and a cross-sectional information image corresponding to the detected cross-sectional information.

The ultrasound diagnostic apparatus may further include a memory that maps and stores the cross-sectional information image corresponding to the cross-sectional information.

The stored cross-sectional information image may be a cross-sectional image corresponding to the ultrasound image.

The cross-sectional information image may be an image in which a cross-sectional surface corresponding to the ultrasound image is displayed in a three-dimensional (3D) image of the object.

The cross-sectional information detecting unit may extract at least one of a shape, length, width, and brightness value of a sub-object included in the ultrasound image and a position relationship with a peripheral sub-object to detect the cross-sectional information of the ultrasound image.

The display unit may display a first pointer at a first position of the ultrasound image, based on a user input, and display a second pointer at a second position of the cross-sectional information image corresponding to a position of the first pointer.

The display unit may display names of a plurality of sub-objects included in the ultrasound image, based on the cross-sectional information image.

The ultrasound image may include first and second ultrasound images, the display unit may display the first and second ultrasound images, and display a first cross-sectional surface corresponding to the first ultrasound image and a second cross-sectional surface corresponding to the second ultrasound image, in a three-dimensional (3D) image of the object.

The display unit may display the first and second cross-sectional surfaces in different colors.

The ultrasound diagnostic apparatus may further include a user input unit that receives a user input for selecting one of the first and second ultrasound images, wherein the display unit may display a cross-sectional image corresponding to the selected ultrasound image, based on the user input.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram illustrating a configuration of an ultrasound diagnostic apparatus according to an embodiment of the present invention;
FIG. 2 is a block diagram illustrating a configuration of an ultrasound diagnostic apparatus according to an embodiment of the present invention;
FIG. 3 is a flowchart illustrating a method of operating an ultrasound diagnostic apparatus according to an embodiment of the present invention; and
FIGS. 4 to 6 are diagrams for explaining the operating method of FIG. 3.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

All terms including descriptive or technical terms which are used herein should be construed as having meanings that are obvious to one of ordinary skill in the art. However, the terms may have different meanings according to an intention of one of ordinary skill in the art, precedent cases, or the appearance of new technologies. Also, some terms may be arbitrarily selected by the applicant, and in this case, the meaning of the selected terms will be described in detail in the detailed description of the invention. Thus, the terms used herein have to be defined based on the meaning of the terms together with the description throughout the specification.

Also, when a part "includes" or "comprises" an element, unless there is a particular description contrary thereto, the part may further include other elements, not excluding the other elements. Moreover, each of terms such as "...unit" and "module" described in specification denotes an element for performing at least one function or operation, and may be implemented in hardware, software or a combination of hardware and software.

The term "ultrasound image" used herein denotes an image of an object acquired by using an ultrasound signal. Also, the term "object" used herein may include a person, an animal, a part of the person, or a part of the animal. For example, an object may include an organ such as a liver, a heart, a womb, a brain, breasts, an abdomen, or the like, or a blood vessel. Also, the term "object" may include a phantom. The phantom denotes a material having a volume that is very close to a density and effective atomic number of an organism, and may include a spherical phantom having a characteristic similar to a physical body.

Moreover, the ultrasound image may be implemented in various ways. For example, the ultrasound image may be at least one of an amplitude (A) mode image, a brightness (B) mode image, a color (C) mode image, and a Doppler (D) mode image. Also, according to an embodiment of the present invention, the ultrasound image may be a two-dimensional (2D) image or a three-dimensional (3D) image.

Moreover, the term "user" used herein is a medical expert, and may be a doctor, a nurse, a medical technologist, a medical image expert, or the like, or may be an engineer who repairs a medical apparatus. However, the user is not limited thereto.

The present invention will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those of ordinary skill in the art. In the following description, well-known functions or constructions are not described in detail since they would obscure the invention with unnecessary detail. Throughout the specification, like reference numerals in the drawings denote like elements.

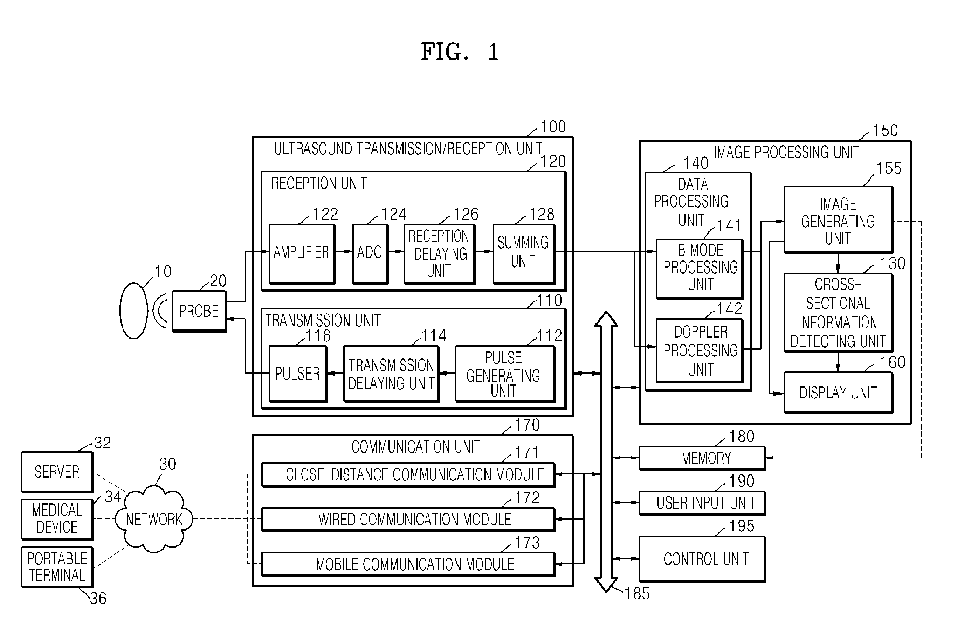

FIG. 1 is a block diagram illustrating a configuration of an ultrasound diagnostic apparatus 100 according to an embodiment of the present invention.

Referring to FIG. 1, the ultrasound diagnostic apparatus 100 according to an embodiment of the present invention includes a probe 20, an ultrasound transceiver 115, an image processor 150, a communicator 170, a memory 180, a user input unit 190, and a controller 195. The above-described elements may be connected to each other through a bus 185. Also, the image processor 150 may include an image generating unit 155, a cross-sectional information detecting unit 130, and a display unit 160.

The ultrasound diagnostic apparatus 100 may be implemented as a portable type as well as a card type. Examples of the portable diagnostic apparatuses may include picture archiving and communication system (PACS) viewers, smartphones, laptop computers, personal digital assistants (PDAs), tablet personal computers (PCs), etc., but are not limited thereto.

The probe 20 transmits ultrasound signals to an object 10 based on a driving signal applied by the ultrasound transceiver 115 and receives echo signals reflected by the object 10. The probe 20 includes a plurality of transducers, and the plurality of transducers oscillate based on electric signals transmitted thereto and generate acoustic energy, that is, ultrasound signals. Furthermore, the probe 20 may be connected to the main body of the ultrasound diagnostic apparatus 100 by wire or wirelessly. According to embodiments of the present invention, the ultrasound diagnostic apparatus 100 may include a plurality of probes 20.

A transmission unit 110 supplies a driving signal to the probe 20 and includes a pulse generating unit 112, a transmission delaying unit 114, and a pulser 116. The pulse generating unit 112 generates pulses for forming transmission ultrasound signals based on a predetermined pulse repetition frequency (PRF), and the transmission delaying unit 114 applies a delay time for determining transmission directionality to the pulses. Pulses to which a delay time is applied correspond to a plurality of piezoelectric vibrators included in the probe 20, respectively. The pulser 116 applies a driving signal (or a driving pulse) to the probe 20 as a timing corresponding to each pulse to which a delay time is applied.

A reception unit 120 generates ultrasound data by processing echo signals received from the probe 20 and may include an amplifier 122, an analog-digital converter (ADC) 124, a reception delaying unit 126, and a summing unit 128. The amplifier 122 amplifies echo signals in each channel, and the ADC 124 analog-to-digital converts the amplified echo signals. The reception delaying unit 126 applies delay times for determining reception directionality to the digital-converted echo signals, and the summing unit 128 generates ultrasound data by summing the echo signals processed by the reception delaying unit 126.

The image processor 150 generates an ultrasound image by scan-converting ultrasound data generated by the ultrasound transceiver 115 and displays the ultrasound image.

An ultrasound image may include not only a grayscale ultrasound image obtained by scanning an object in an amplitude (A) mode, a brightness (B) mode, and a motion (M) mode, but also a blood flow Doppler image showing flow of blood (also referred to as a color Doppler image), a tissue Doppler image showing movement of tissues, and a spectral Doppler image showing moving speed of an object as a waveform.

A B mode processor 141 extracts B mode components from ultrasound data and processes the B mode components. An image generating unit 155 may generate an ultrasound image indicating signal intensities as brightness based on the extracted B mode components.

Similarly, a Doppler processor 142 may extract Doppler components from ultrasound data, and the image generating unit 155 may generate a Doppler image indicating movement of an object as colors or waveforms based on the extracted Doppler components.

The image generating unit 155 according to an embodiment of the present invention may generate a 2D ultrasound image via volume-rendering of volume data and may also generate an elasticity image which visualizes deformation of an object 10 due to pressure. Furthermore, the image generating unit 155 may display various additional information in an ultrasound image by using texts and graphics. The generated ultrasound image may be stored in the memory 180.

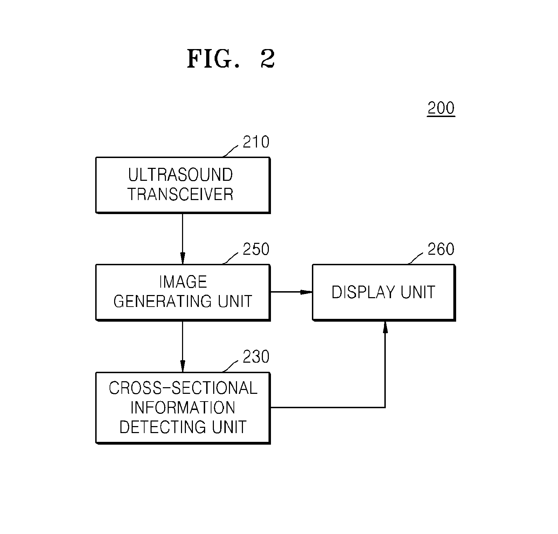

The cross-sectional information detecting unit 130 may detect cross-sectional information indicating which cross-sectional surface of the object 10 an ultrasound image shows, on the basis of the ultrasound image generated by the image generating unit 155. This will be described in detail with reference to FIG. 2.

The display unit 160 displays the ultrasound image generated by the image generating unit 155. The display unit 160 may display various pieces of information processed by the ultrasound diagnostic apparatus 100, in addition to the ultrasound image, on a screen through a graphics user interface (GUI). The ultrasound diagnostic apparatus 100 may include two or more display units 160 depending on an implementation type.

The display unit 160 includes at least one of a liquid crystal display (LCD), a thin film transistor-liquid crystal display (TFT-LCD), an organic light-emitting diode (OLED), a flexible display, a 3D display, and an electrophoretic display.

Moreover, when the display unit 160 and the user input unit 190 are implemented as a touch screen by forming a layer structure, the display unit 160 may be used as an input unit that enables information to be input by a user's touch, in addition to an output unit.

The touch screen may be configured to detect a touch pressure in addition to a touch input position and a touched area. Also, the touch screen may be configured to detect a proximity touch as well as a real touch.

Herein, the term "real touch" denotes a case in which a pointer really touches a screen, and the term "proximity touch" denotes a case in which the pointer does not actually touch the screen but approaches a position which is separated from the screen by a certain distance. The pointer used herein denotes a touch instrument for really touching or proximity-touching a specific portion of a displayed screen. Examples of the pointer include an electronic pen, a finger, etc.

Although not shown, the ultrasound diagnostic apparatus 100 may include various sensors inside or near the touch screen, for detecting a real touch or a proximity touch on the touch screen. An example of a sensor for sensing a touch of the touch screen is a tactile sensor.

The tactile sensor denotes a sensor that senses a touch by a specific object to a degree to which a user feels, or more. The tactile sensor may sense various pieces of information such as a roughness of a touched surface, a stiffness of a touched object, a temperature of a touched point, etc.

Moreover, an example of a sensor for sensing a touch of the touch screen is a proximity sensor. The proximity sensor denotes a sensor that detects an object approaching a detection surface or an object near the detection surface by using an electromagnetic force or infrared light without any mechanical contact.

Examples of the proximity sensor include a transmissive photosensor, a directly reflective photosensor, a mirror reflective photosensor, a high frequency oscillation-type proximity sensor, a capacitive proximity sensor, a magnetic proximity sensor, and an infrared proximity sensor.

The communicator 170 is connected to a network 30 in a wired or wireless manner to communicate with an external device or server. The communicator 170 may exchange data with a hospital server or a medical apparatus of a hospital which is connected thereto through a medical image information system (a PACS). Also, the communicator 170 may perform data communication according to the digital imaging and communications in medicine (DICOM) standard.

The communicator 170 may transmit and receive data, such as an ultrasound image, ultrasound data, Doppler data, etc. of an object, associated with a diagnosis of the object over the network 30, and may also transmit and receive a medical image captured by a medical apparatus such as a computed tomography (CT) apparatus, a magnetic resonance imaging (MRI) apparatus, or an X-ray apparatus. Furthermore, the communicator 170 may receive information on a diagnosis history or treatment schedule of a patient from a server, and use a diagnosis of an object. In addition, the communicator 170 may perform data communication with a portable terminal of a doctor or a patient, in addition to a server or medical apparatus of a hospital.

The communicator 170 may be connected to the network 30 in a wired or wireless manner, and may exchange data with a server 32, a medical apparatus 34, or a portable terminal 36. The communicator 170 may include one or more elements that enable communication with an external device, and for example, include a short-distance communication module 171, a wired communication module 172, and a mobile communication module 173.

The short-distance communication module 171 denotes a module for short-distance communication within a certain distance. Short-distance communication technology, according to an embodiment of the present invention, may include wireless LAN, Wi-Fi, Bluetooth, Zigbee, Wi-Fi direct (WFD), ultra wideband (UWB), infrared data association (IrDA), Bluetooth low energy (BLE), and near field communication (NFC), but the short-distance communication technology is not limited thereto.

The wired communication module 172 denotes a module for communication using an electrical signal or an optical signal. Wired communication technology according to an embodiment may include a pair cable, a coaxial cable, an optical fiber cable, or an Ethernet cable.

The mobile communication module 173 transmits and receives a radio frequency (RF) signal to and from a base station, an external terminal, and a server over a mobile communication network. Here, the RF signal may include various types of data based on transmission and reception of a voice call signal, a video call signal, or a letter/multimedia message.

The memory 180 stores various pieces of information processed by the ultrasound diagnostic apparatus 100. For example, the memory 180 may store medical data, such as input/output ultrasound data and ultrasound images, associated with a diagnosis of an object, and may also store an algorithm or a program which is executed in the ultrasound diagnostic apparatus 100.

According to an embodiment of the present invention, the memory 180 may store a previously-mapped cross-sectional information image corresponding to cross-sectional information of an object. For example, the memory 180 may store a first cross-sectional information image corresponding to first cross-sectional information and a second cross-sectional information image corresponding to second cross-sectional information. The cross-sectional information may include various pieces of data for analyzing a cross-sectional surface of the object. For example, the first cross-sectional information may include data of a shape, length, or width of the sub-object, which is included in an ultrasound image of a first cross-sectional surface of the object, and a brightness value (which is shown in only the first cross-sectional image) within a certain range.

The memory 180 may be configured with various kinds of storage mediums such as a flash memory, a hard disk, an EEPROM, etc. Also, the ultrasound diagnostic apparatus 100 may operate web storage or a cloud server which performs a storage function of the memory 180 on a web.

The user input unit 190 generates input data which is input by a user for controlling an operation of the ultrasound diagnostic apparatus 100. The user input unit 190 may include hardware elements such as a keypad, a mouse, a touch pad, a trackball, a jog switch, but is not limited thereto. As another example, the user input unit 190 may further include various sensors such as an electrocardiogram (ECG) measurement module, a breath measurement sensor, a voice recognition sensor, a gesture recognition sensor, a fingerprint recognition sensor, an iris recognition sensor, a depth sensor, a distance sensor, etc.

In particular, the user input unit 190 may further include the touch screen in which the touch pad and the display unit 160 form the layer structure.

In this case, the ultrasound diagnostic apparatus 100 may display a specific mode ultrasound image and a control panel for an ultrasound image, on the touch screen. In addition, the ultrasound diagnostic apparatus 100 may sense a user's touch gesture for an ultrasound image through the touch screen.

The ultrasound diagnostic apparatus 100 according to an embodiment of the present invention may physically include some buttons, frequently used by a user, from among a plurality of buttons included in a control panel of general ultrasound diagnostic apparatuses, and the other buttons may be provided through a type of GUI on the touch screen.

The controller 195 controls an overall operation of the ultrasound diagnostic apparatus 100. That is, the controller 195 may control operations between the probe 20, the ultrasound transceiver 115, the image processor 150, the communicator 170, the memory 180, and the user input unit 190 which are illustrated in FIG. 1.

Some or all of the probe 20, the ultrasound transceiver 115, the image processor 150, the communicator 170, the memory 180, the user input unit 190, and the controller 195 may be operated by a software module, but are not limited thereto. Some of the above-described elements may be operated by a hardware module. Also, at least some of the ultrasound transceiver 115, the image processor 150, and the communicator 170 may be included in the controller 195, but are not limited to the implementation type.

FIG. 2 is a block diagram illustrating a configuration of an ultrasound diagnostic apparatus 200 according to an embodiment of the present invention. Referring to FIG. 2, the ultrasound diagnostic apparatus 200 may include an ultrasound transceiver 210, an image generating unit 250, a cross-sectional information detecting unit 230, and a display unit 260.

The ultrasound transceiver 210 of FIG. 2 is an element corresponding to the ultrasound transceiver 115 of FIG. 1, the image generating unit 250 of FIG. 2 is an element corresponding to the image generating unit 155 of FIG. 1, the cross-sectional information detecting unit 230 of FIG. 2 is an element corresponding to the cross-sectional information detecting unit 130 of FIG. 1, and the display unit 260 of FIG. 2 is an element corresponding to the display unit 160 of FIG. 1. Thus, the same descriptions are not repeated.

The image generating unit 250 may generate a 2D ultrasound image by using ultrasound data which corresponds to a received echo signal.

The cross-sectional information detecting unit 230 may detect cross-sectional information on the basis of the ultrasound image, and determine which cross-sectional surface of an object the ultrasound image shows, on the basis of the cross-sectional information.

For example, the cross-sectional information may be at least one of a shape, length, width, and brightness value of a sub-object included in the ultrasound image and a position relationship with respect to a peripheral sub-object. The cross-sectional information detecting unit 230 may compare detected cross-sectional information with cross-sectional information stored in a memory to analyze which cross-sectional surface of the object the ultrasound image shows.

In this case, the memory 180 may store a cross-sectional image, indicating a cross-sectional surface of the object, and cross-sectional information corresponding to the cross-sectional image. For example, when the object is a heart, the memory 180 may store a parasternal view image, indicating a parasternal view of the heart, and parasternal view information (for example, data of a shape, length, width, and brightness value of a sub-object shown in only the parasternal view image and a position relationship with respect to a peripheral sub-object), corresponding to the parasternal view image, to be mapped to each other. Also, the memory 180 may store an apical view image, indicating an apical view of the heart, and apical view information, corresponding to the apical view image, to be mapped to each other. However, the present embodiment is not limited thereto, and the memory 180 may store cross-sectional information and a cross-sectional image, indicating each of a plurality of cross-sectional surfaces of the object, to be mapped to each other.

Alternatively, the cross-sectional information detecting unit 230 may detect a direction and angle of a probe that transmits an ultrasound signal to determine which cross-sectional surface of an object an ultrasound image shows. For example, the cross-sectional information detecting unit 230 may detect an inclined angle and rotational angle of the probe to determine a position of a cross-sectional surface corresponding to the ultrasound image.

The display unit 260 may display the ultrasound image and a cross-sectional image corresponding to the ultrasound image. Also, the display unit 260 may display a cross-sectional information image indicating a position of a cross-sectional surface corresponding to the ultrasound image in the object.

For example, the display unit 260 may display a cross-sectional image that matches cross-sectional information detected by the cross-sectional information detecting unit 230. Also, the display unit 260 may display a three-dimensional (3D) image indicating the object and a cross-sectional surface corresponding to the ultrasound image in order for the cross-sectional surface to overlap on the 3D image.

Moreover, the display unit 260 may display the cross-sectional image corresponding to the ultrasound image. For example, when cross-sectional information of the ultrasound image detected by the cross-sectional information detecting unit 230 matches the parasternal view information stored in the memory, the display unit 260 may display the parasternal view information stored in the memory.

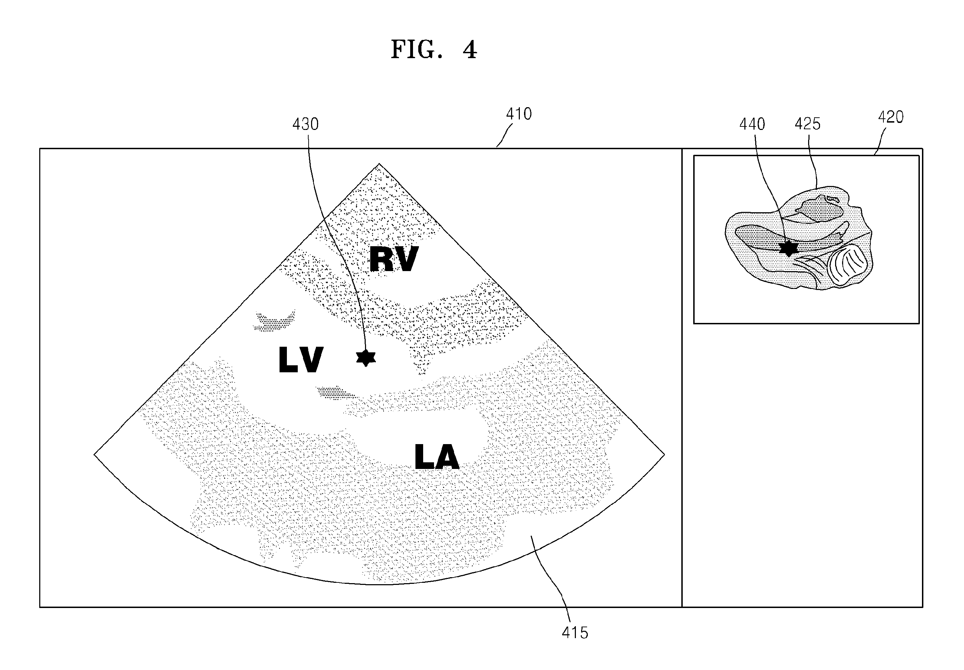

An operation of the display unit 260 will be described in detail with reference to FIGS. 4 to 6.

The block diagram of each of the ultrasound diagnostic apparatuses 100 and 200 of FIGS. 1 and 2 is a block diagram according to an embodiment of the present invention. The elements of the block diagram may be integrated, added, or omitted depending on a specification of an actually implemented cache memory system. That is, depending on the case, two or more elements may be integrated into one element, or one element may be subdivided into two or more elements. Also, a function performed by each element is for describing an embodiment of the present invention, and each element or a detailed operation thereof does not limit the scope and spirit of the present invention.

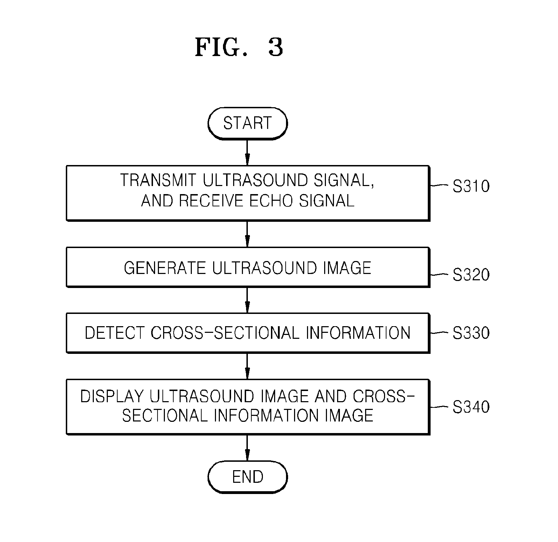

FIG. 3 is a flowchart illustrating a method of operating an ultrasound diagnostic apparatus according to an embodiment of the present invention.

Referring to FIG. 3, the ultrasound diagnostic apparatus 100 (200) may transmit an ultrasound signal to an object, and receive an echo signal reflected from the object in operation S310.

Hereinafter, for convenience of description, a case in which the object is a heart will be described as an example. However, the present embodiment is not limited thereto.

The ultrasound diagnostic apparatus 100 (200) may generate an ultrasound image on the basis of the received echo signal in operation S320.

For example, the ultrasound diagnostic apparatus 100 (200) may process the received echo signal to generate ultrasound data, and generate an ultrasound image of the object on the basis of the generated ultrasound data. Here, the ultrasound image may be a 2D image indicating a cross-sectional surface of the object. Also, as illustrated in FIG. 4, the ultrasound image may be a B mode image, but is not limited thereto.

In operation S330, the ultrasound diagnostic apparatus 100 (200) may detect cross-sectional information indicating which cross-sectional surface of the object the generated ultrasound image shows.

For example, the ultrasound diagnostic apparatus 100 (200) may detect information about a shape, length, width, and brightness value of a sub-object included in the ultrasound image and a position relationship with respect to a peripheral sub-object, and compare detected information with cross-sectional information stored in a memory to analyze which cross-sectional surface of the object the ultrasound image shows.

Alternatively, the ultrasound diagnostic apparatus 100 (200) may detect a direction and angle of a probe that transmits the ultrasound signal to determine which cross-sectional surface of an object an ultrasound image shows. For example, the ultrasound diagnostic apparatus 100 (200) may detect an inclined angle and rotational angle of the probe to determine a position of a cross-sectional surface corresponding to the ultrasound image.

In operation S340, the ultrasound diagnostic apparatus 100 (200) may display the ultrasound image and a cross-sectional information image corresponding to the detected cross-sectional information.

For example, referring to FIG. 4, the display unit 160 (260) may include a first region and a second region. Here, the display unit 160 (260) may display an ultrasound image 415 in the first region, and display a cross-sectional information image 425 in the second region 420.

In this case, the ultrasound image 415 displayed in the first region may be a 2D ultrasound image of the object, and may be a B mode image. Also, names of sub-objects included in the ultrasound image may be displayed to overlap on the ultrasound image 415. For example, as illustrated in FIG. 4, when the ultrasound image 415 is a 2D ultrasound image of a heart, the ultrasound diagnostic apparatus 100 (200) may detect an object such as a left ventricle (LV), a right ventricle (RV), a left atrium (LA), or a right atrium (RA), and may display a corresponding name to overlap on the ultrasound image 415.

The cross-sectional information image 425, indicating which cross-sectional surface of the object the ultrasound image 415 displayed in the first region 410 shows, may be displayed in the second region 420. Here, the cross-sectional information image 425 may be an image of a certain cross-sectional surface of the object, and may be an image stored in the memory.

Referring again to FIG. 4, on the basis of a user input, a moving first pointer 430 may be displayed in the ultrasound image 415 displayed in the first region 410, and a second pointer 440 may be displayed at coordinates (corresponding to first pointer coordinates) of the cross-sectional information image 425 displayed in the second region 420.

A cross-sectional information image, indicating a position of a cross-sectional surface 530 corresponding to an ultrasound image 515 displayed in a first region 530, may be displayed in a second region 520 of the display unit 160 (260).

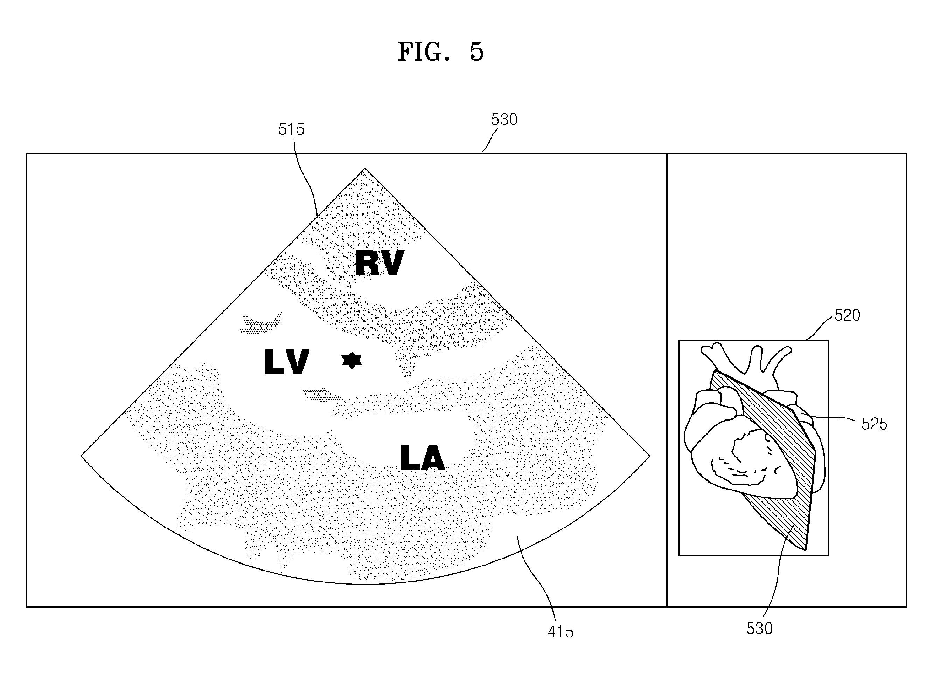

For example, referring to FIG. 5, a 3D image 525 of an object may be displayed in the second region 520, and the cross-sectional surface 530 corresponding to the ultrasound image 515 displayed in the first region 510 may be displayed to overlap the 3D image 525. Here, the 3D image 525 may be a 3D modeling image of the object, and an overlapped cross-sectional surface 530 may be displayed as slashes or may be highlighted.

The cross-sectional information image (a 3D image with a cross-sectional surface displayed therein) displayed in the second region 520 may indicate in which direction the ultrasound image 515 displayed in the first region 510 is an ultrasound image of a surface of the object. Therefore, a user may easily determine in which direction the ultrasound image 515 displayed in the first region 510 is an ultrasound image of a surface of the object, while looking at the cross-sectional information image displayed in the second region 520, and may adjust an angle and position of the probe 20 to obtain an appropriate cross-sectional ultrasound image.

Moreover, the ultrasound diagnostic apparatus 100 (200) may rotate, in various directions, the 3D image 525 with a cross-sectional surface displayed therein on the basis of a user input. Therefore, the user may easily determine a position of the cross-sectional surface while rotating the 3D image 525.

Moreover, the ultrasound diagnostic apparatus 100 (200) may move the cross-sectional surface displayed in the 3D image 525 on the basis of the user input, and an ultrasound image corresponding to the moved cross-sectional surface may be displayed in the first region 510.

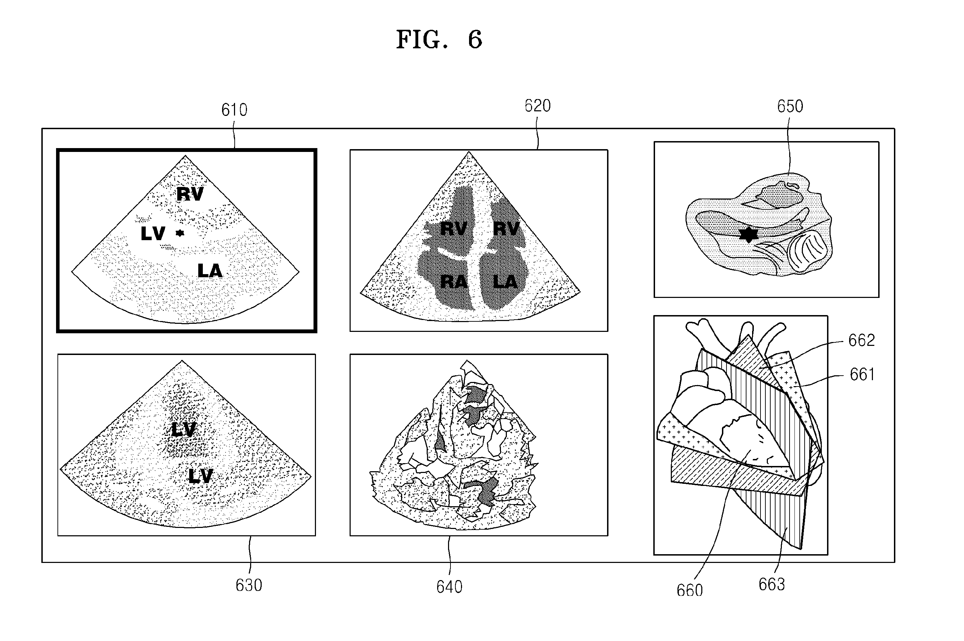

The ultrasound diagnostic apparatus 100 (200) may display a plurality of ultrasound images. Referring to FIG. 6, the ultrasound diagnostic apparatus 100 (200) may display a cross-sectional ultrasound image (a first ultrasound image 610) of an object in a first direction, a cross-sectional ultrasound image (a second ultrasound image 620) of the object in a second direction, and a cross-sectional ultrasound image (a third ultrasound image 630) of the object in a third direction.

Moreover, the ultrasound diagnostic apparatus 100 (200) may display a 3D image 640 which is generated on the basis of a 2D ultrasound image of the object. For example, the ultrasound diagnostic apparatus 100 (200) may generate and display the 3D image 640 by using the first to third ultrasound images 610, 620 and 630.

Moreover, the ultrasound diagnostic apparatus 100 (200) may display a cross-sectional information image indicating a position of a cross-sectional surface corresponding to each of the first to third ultrasound images 610, 620 and 630 in the object. For example, the ultrasound diagnostic apparatus 100 (200) may display cross-sectional surfaces 661 to 663, respectively corresponding to the first to third ultrasound images 610, 620 and 630, in a 3D image 660 of the object to overlap each other.

In this case, a first cross-sectional surface 661 corresponding to the first ultrasound image 610, a second cross-sectional surface 662 corresponding to the second ultrasound image 620, and a third cross-sectional surface 663 corresponding to the third ultrasound image 630 may be displayed in different colors to be distinguished.

Moreover, when a user input for selecting one of the first to third cross-sectional surfaces 661 to 663 is received, the ultrasound diagnostic apparatus 100 (200) may highlight and display an ultrasound image corresponding to a selected cross-sectional surface.

Moreover, the ultrasound diagnostic apparatus 100 (200) may receive a user input for selecting one of the plurality of ultrasound images displayed by the display unit 160 (260), and display a cross-sectional image corresponding to the selected ultrasound image.

For example, when a user input for selecting the first ultrasound image 610 from among the first to third ultrasound images 610, 620 and 630 is received, the first ultrasound image 610 may be highlighted and displayed for indicating the selection of the first ultrasound image 610, and the display unit 160 (260) may display a cross-sectional image 650 corresponding to the first ultrasound image 610. Here, the cross-sectional image 650 may be an image stored in the memory.

Moreover, the first cross-sectional surface 661 which is displayed to overlap the 3D image of the object may be highlighted and displayed, thereby informing the user that a cross-sectional surface corresponding to the selected first ultrasound image 610 is the first cross-sectional surface 661.

As described above, according to the one or more of the above embodiments of the present invention, which cross-sectional surface of an object a displayed ultrasound image shows is easily determined, and thus, an object may be accurately diagnosed.

The ultrasound diagnostic apparatus and the method of operating the same according to the present invention may also be embodied as computer readable codes on a computer readable recording medium. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, and optical data storage. The computer readable recording medium can also be distributed over network coupled computer systems so that the computer readable code may be stored and executed in a distributed fashion.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

## Claims

1. A method of operating an ultrasound diagnostic apparatus, the method comprising:
transmitting an ultrasound signal to an object to receive an echo signal corresponding to the ultrasound signal from the object;
generating an ultrasound image, based on the received echo signal;
detecting cross-sectional information indicating which cross-sectional surface of the object the generated ultrasound image shows; and
displaying the ultrasound image and a cross-sectional information image corresponding to the detected cross-sectional information.

2. The method of claim 1, further comprising mapping and storing the cross-sectional information image corresponding to the cross-sectional information.

3. The method of claim 1, wherein the detecting of the cross-sectional information comprises extracting at least one of a shape, length, width, and brightness value of a sub-object included in the ultrasound image and a position relationship with respect to a peripheral sub-object to detect the cross-sectional information of the ultrasound image.

4. The method of claim 1, further comprising:
displaying a first pointer at a first position of the ultrasound image, based on a user input; and
displaying a second pointer at a second position of the cross-sectional information image corresponding to a position of the first pointer.

5. The method of claim 1, further comprising displaying names of a plurality of sub-objects included in the ultrasound image, based on the cross-sectional information image.

6. The method of claim 1, wherein,
the ultrasound image comprises first and second ultrasound images,
the displaying of the ultrasound image comprises displaying the first and second ultrasound images, and
the displaying of the cross-sectional information image comprises displaying a first cross-sectional surface corresponding to the first ultrasound image and a second cross-sectional surface corresponding to the second ultrasound image, in a 3D image of the object.

7. The method of claim 6, further comprising selecting one of the first and second ultrasound images, based on a user input,
wherein the displaying of the cross-sectional information image comprises displaying a cross-sectional image which corresponds to the selected ultrasound image.

8. An ultrasound diagnostic apparatus comprising:
an ultrasound transceiver that transmits an ultrasound signal to an object, and receives an echo signal corresponding to the ultrasound signal from the object;
an image generating unit that generates an ultrasound image, based on the received echo signal;
a cross-sectional information detecting unit that detects cross-sectional information indicating which cross-sectional surface of the object the generated ultrasound image shows; and
a display unit that displays the ultrasound image and a cross-sectional information image corresponding to the detected cross-sectional information.

9. The ultrasound diagnostic apparatus of claim 8, further comprising a memory that maps and stores the cross-sectional information image corresponding to the cross-sectional information.

10. The ultrasound diagnostic apparatus of claim 8, wherein the cross-sectional information image is an image in which a cross-sectional surface corresponding to the ultrasound image is displayed in a three-dimensional (3D) image of the object.

11. The ultrasound diagnostic apparatus of claim 8, wherein the cross-sectional information detecting unit extracts at least one of a shape, length, width, and brightness value of a sub-object included in the ultrasound image and a position relationship with respect to a peripheral sub-object to detect the cross-sectional information of the ultrasound image.

12. The ultrasound diagnostic apparatus of claim 8, wherein the display unit displays a first pointer at a first position of the ultrasound image, based on a user input, and displays a second pointer at a second position of the cross-sectional information image corresponding to a position of the first pointer.

13. The ultrasound diagnostic apparatus of claim 8, wherein the display unit displays names of a plurality of sub-objects included in the ultrasound image, based on the cross-sectional information image.

14. The ultrasound diagnostic apparatus of claim 8, wherein,
the ultrasound image comprises first and second ultrasound images,
the display unit displays the first and second ultrasound images, and displays a first cross-sectional surface corresponding to the first ultrasound image and a second cross-sectional surface corresponding to the second ultrasound image, in a three-dimensional (3D) image of the object.

15. The ultrasound diagnostic apparatus of claim 14, further comprising a user input unit that receives a user input for selecting one of the first and second ultrasound images,
wherein the display unit displays a cross-sectional image corresponding to the selected ultrasound image, based on the user input.
